# EUROPÄISCHE PATENTSCHRIFT

(11) **EP 2 237 834 B1**
(45) Veröffentlichungstag und Bekanntmachung des Hinweises auf die Patenterteilung: **18.01.2012**
(21) Anmeldenummer: 09706888.6
(22) Anmeldetag: 23.01.2009
(51) Int. Cl.: A61N 1/36

(54) **VORRICHTUNG ZUR ELEKTROTHERAPEUTISCHEN BEHANDLUNG VON MUSKEL- UND NERVENGEWEBE**
DEVICE FOR ELECTROTHERAPEUTIC TREATMENT OF MUSCLE AND NERVE TISSUE
DISPOSITIF DE TRAITEMENT ELECTROTHERAPEUTIQUE DE TISSUS MUSCULAIRES ET NERVEUX

(30) Priorität: 31.01.2008 DE 102008007063
(43) Veröffentlichungstag der Anmeldung: 13.10.2010
(73) Patentinhaber: May, Renate, 75015 Bretten (DE); May, Hans-Ulrich, 75015 Bretten (DE)
(72) Erfinder: MAY, Hans-Ulrich, 75015 Bretten (DE)
(74) Vertreter: Geitz Truckenmüller Lucht
(86) Internationale Anmeldenummer: PCT/DE2009/075000
(87) Internationale Veröffentlichungsnummer: WO 2009/095013

(56) Entgegenhaltungen:
- DE-A1- 10 039 240
- FR-A3- 2 500 309
- ANONYMOUS: "HiToP-Einführung in die Hochtontherapie" GBO MEDIZINTECHNIK AG PROSPEKT, [Online] Mai 2007 (2007-05), Seiten 1-24, XP002545363 RIMBACH Gefunden im Internet: URL:http://www.gbo-medizintechnik.de/de/pd f/Prospekt_Einfuehrung_Hochtontherapie.pdf > [gefunden am 2009-09-10]
- ANONYMOUS: "HiToP-Therapiegerät: HiToP 191 Bedienungsanleitung" GBO MEDIZINTECHNIK AG HANDBUCH, [Online] 2004, XP002545365 RIMBACH Gefunden im Internet: URL:http://www.gbo-medizintechnik.de/de/pd f/bedienungsanleitungen/BH191_DT.pdf> [gefunden am 2009-09-10]

## Beschreibung

Die vorliegende Erfindung betrifft eine Vorrichtung zur elektrotherapeutischen Behandlung von Muskel- und Nervengewebe, mit einem Signalgeber zum Generieren von Wechselstrom definierter Frequenz und Amplitude sowie wenigstens zwei Elektroden zur Beaufschlagung des Muskel- und Nervengewebes mit diesem Wechselstrom, wobei der Signalgeber periodisch eine simultane Modulation von Frequenz und Amplitude in einer Phase ansteigender Frequenz und einer Phase absteigender Frequenz erzeugt.

Eine solche Vorrichtung ist aus der DE 100 39 240 A1 vorbekannt. Diese Schrift betrifft ein elektrotherapeutisches Gerät zur Behandlung etwa des menschlichen Körpers durch Beaufschlagung desselben mit Wechselstrom mit simultan modulierter Frequenz und Amplitude.

Für die Auslösung von Reizwirkungen in den behandelten Muskel- und Nervengeweben sind Amplitude und Frequenz des angelegten Stromes sowie ihr Verhältnis zueinander maßgebend. Eine Reizwirkung wird dabei lediglich oberhalb einer Reizschwelle bewirkt, an welcher die Verläufe von Frequenz und Amplitude auszurichten sind. Innerhalb des Amplituden- und Frequenzverlaufs während der Behandlung ist zwischen einer Phase ansteigender Frequenz und einer Phase absteigender Frequenz zu unterscheiden, wobei jeweils mitzulesen ist, daß eine simultane Modulation der Amplitude jeweils entsprechend erfolgt.

Die aufsteigende Phase führt im Laufe der Anwendung zu Reizzwecken von der Überschwelligkeit bezüglich der Reizschwelle in die Unterschwelligkeit, d.h. der etwa bei 4 kHz maximal überschwellig gewordenen Modulation wird durch in Vierteltonschritten erfolgende Frequenzzunahmen und simultan erfolgende Intensitäts- Zu- oder Abnahmen ein "Ausschleichvorgang" realisiert, der bei Muskelreizungen zu einer allmählichen und nicht abrupten Erschlaffung der gereizten Muskeln führt.

Die absteigende Phase führt umgekehrt von der Unterschwelligkeit in die Überschwelligkeit, bewirkt also die eigentliche Reizung des behandelten Gewebes.

Das Behandlungsfrequenzband der Trägerfrequenz umfasst insgesamt 3 Oktaven, beginnend mit einer Frequenz von 2¹² Hz (also 4096 Hz oder 4kHz). Eine Erhöhung um eine Oktave bedeutet bekanntermaßen eine Verdopplung der Frequenz, so daß das Band bis 2¹⁵ Hz (also 32768 Hz oder 32 kHz) reicht. Bevorzugtermaßen wird die Frequenz in Vierteltonschritten erhöht, so daß einschließlich der Oktavengrenzen selbst insgesamt 73 Trägerfrequenzen im Rahmen der Behandlung angeboten werden können.

Bei höheren Modulationsfrequenzen entsteht jedoch das Problem, daß in der für höher werdende Modulationsfrequenzen immer kürzer werdenden, zur Frequenz sich reziprok verhaltenden Periodendauern nicht mehr alle 73 Trägerfrequenzen wenigstens zweimal, also für wenigstens zwei Perioden der Trägerfrequenz, angeboten werden können. Dies wäre wünschenswert, um die Frequenzen der aufsteigenden und der absteigenden Phase lückenlos zu durchlaufen.

Beträgt zum Beispiel die Modulationsfrequenz 20 Hz, so hat diese eine Periodendauer von 1/20 s, also 50 ms, demnach 25 ms für die ansteigende und 25 ms für die absteigende Phase. Sollen nunmehr die 73 Mittelfrequenzen in diesen 25 ms untergebracht werden, so stehen für jede Mittelfrequenz nur 0,34 ms zur Verfügung, bei einer Trägerfrequenz von 4 kHz sind dies noch nicht einmal zwei Perioden, bei 32 kHz immerhin etwa elf Perioden. Das Problem verschärft sich bei weiterer Zunahme der Modulationsfrequenz.

Vor diesem Hintergrund liegt der vorliegenden Erfindung die Aufgabe zu Grunde, eine lückenlose Behandlung in den bevorzugten Frequenzbändern zu ermöglichen.

Gelöst wird diese Aufgabe durch eine Vorrichtung gemäß den Merkmalen des Hauptanspruchs. Weitere sinnvolle Ausgestaltungen können den Unteransprüchen entnommen werden.

Erfindungsgemäß wird zur Erzielung einer hinsichtlich der Abdeckung des Frequenzbands lückenlosen Behandlung die Phase ansteigender Frequenz verkürzt, so daß die Phase absteigender Frequenz entsprechend verlängert werden kann. Während das Absteigen der Frequenz eine Muskelkontraktion bewirkt, verursacht eine ansteigende Frequenz die Muskelerschlaffung. Im Gegensatz zu einer beschleunigten Muskelreizung und der damit verbundenen Kontraktion ist eine abrupte Muskelerschlaffung sinnesphysiologisch, also hinsichtlich der Empfindung, unproblematisch ist und kann folglich in Kauf genommen werden. Die Muskelkontraktion sollte hingegen "sanfter", sukzessive erfolgen. Mit dem Verzicht auf die aufsteigende Modulationsphase kann dies erreicht werden, denn hierdurch werden Zeitfenster für Vierteltonschritte in der absteigenden Phase frei.

Bei dem Übergang von einer nicht wahrnehmbaren Behandlung zu einer Behandlung, welche zur Reizung führt, wird eine so genannte Reizschwelle überschritten, welche beim Durchlaufen der Frequenzen durchkreuzt werden kann. Dies erfolgt bei ansteigender Frequenz von einem überschwelligen Bereich hin zum unterschwelligen Bereich und bei absteigender Frequenz umgekehrt. Besonders angenehm erscheint die Reizung dann, wenn die Reizschwelle von der Frequenz-Amplituden-Kennlinie in spitzem Winkel geschnitten wird.

In einer vorteilhaften Ausgestaltung ist die Frequenz-Amplituden-Kennlinie einer Gerade und somit die Frequenz proportional zur Amplitude gewählt.

Die Vorrichtung stellt, einen Bereich der Modulationsfrequenz von 0,1 Hz bis 200 Hz bereit. Vorzugsweise wird eine Modulationsfrequenz von 20 Hz gewählt, da diese Frequenz mit der natürlichen, im menschlichen Körper zu einer maximal kräftigen und nicht oder erst relativ spät zur Ermüdung führenden Kontraktion eingesetzten Frequenz übereinstimmt. Die modulierte Frequenz, also die Trägerfrequenz, umfasst drei Oktaven von 4 kHz bis 32 kHz, welche bevorzugter Maßen während der Behandlung in diskreten Vierteltonschritten durchlaufen werden.

Dabei wird jeder der genannten Vierteltonschritte zweimal in jeder Periode angeboten.

Die eingekoppelte Leistung ist mit Vorteil nach oben begrenzt, um eine Gefährdung durch zu große Wärme bzw. zu hohe Stromstärken zu verhindern. Insoweit ist die simultan zur Frequenz modulierte Amplitude derart zu begrenzen, daß der Wert der eingekoppelten Leistung 5000 mW nicht übersteigt.

Mithin sind mit der Vorrichtung nicht lediglich einzelne Frequenz-Amplituden-Kennlinien ausführbar; vielmehr ist eine Programmierung der Vorrichtung dahingehend ermöglicht, daß eine Behandlung mithilfe des Geräts nach den Vorgaben und Parametern des Bedieners durchgeführt wird.

Hierzu ist es beispielsweise möglich, die Steigung der Frequenz-Amplituden-Kennlinie zu verändern, so daß die Reizschwelle in unterschiedlichen Winkeln geschnitten werden kann. Diese Kennlinien können dann nacheinander durchlaufen werden, beispielsweise um die Intensität der Behandlung sukzessive zu erhöhen.

Mit Vorteil können zwischen solche Reizphasen auch Pausen eingeschoben werden, welche entweder durch Zurückgehen der Intensität auf Null, oder aber durch Festhalten der Amplitude, vorzugsweise der maximalen Amplitude, bei der oberen Eckfrequenz erreicht werden können. Die Pause wird dadurch nicht "verschenkt", sondern zur maximalen, dabei aber unterschwellig bleibenden Einkopplung von elektrischer Leistung genutzt, z.B. zur Erleichterung von Diffusionsvorgängen.

Der programmierte Therapiervorgang ist mit Vorteil so angelegt, daß ein stetiger Übergang von der "paradoxen" Stimulation (Steigung positiv) über die "synergistische" Stimulation (Steigung negativ) bis zur reinen "vertikalen" Stimulation möglich ist. Dies kann bei Reizung größerer Muskelgruppen oder ganzer Extremitäten wegen der geforderten Limitierung des Outputs auf 5000 mW erforderlich sein, weil ansonsten Leistung im unterschwelligen Bereich reizunwirksam "verschenkt" wird.

Eine vertikale Stimulation sollte mit konstant bleibender oder niederfrequent modulierter Modulationsfrequenz und in Vierteltonschritten nur absteigend oder auch aufsteigend modulierbarer Mittelfrequenz durchgeführt werden. Diese Form der mit höheren Frequenzen als 20 Hz jeweils nur verhältnismäßig kurz und mit angemessenen Pausen durchzuführenden Reizung erscheint für den Aufbau schneller, rasch ermüdender Muskelfasern als eine sinnvolle Ergänzung bisher bekannter Anwendungen.

Die vorstehend beschriebene Erfindung wird nachfolgend anhand eines Ausführungsbeispiels näher erläutert.

Es zeigen
- Figur 1: eine mögliche Frequenz-Amplituden-Kennlinienschar mit einem Sammelpunkt im unterschwelligen Bereich,
- Figur 2: eine mögliche Frequenz-Amplituden-Kennlinienschar mit einem Sammelpunkt im überschwelligen Bereich,
- Figur 3: eine über drei Perioden hinweg dargestellte Frequenz-Amplituden-Kennlinie,
- Figur 4: die Frequenz-Amplituden-Kennlinie aus Figur 3 in einem dreidimensionalen Koordinatensystem, sowie
- Figur 5: eine modulierte Kennlinie.

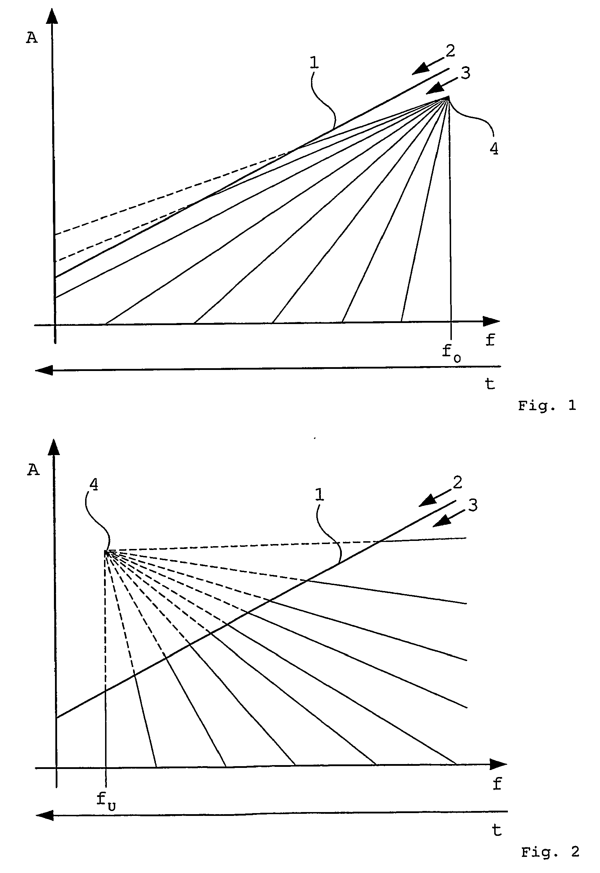

Figur 1 zeigt eine Kurvenschar bestehend aus einer Reihe von Frequenz-Amplituden-Kennlinien, welche in einem Sammelpunkt 4 zusammentreffen. Die Kennlinien verkörpern dabei jeweils einen Durchgang eines Frequenzlaufs, der zur Behandlung von Muskel- und Nervengewebe mithilfe von Wechselstrom mithilfe einer entsprechenden Vorrichtung vorgesehen ist. Die einzelnen Frequenz-Amplituden-Kennlinien werden jeweils einmal in absteigender Frequenzrichtung in diskreten Vierteltonschritten zwischen einer unteren Eckfrequenz f_{U}, welche mit 4 kHz gewählt ist, und einer oberen Eckfrequenz f_{O}, welche mit 32 kHz gewählt ist, durchlaufen. Zwischen den beiden Eckfrequenzen f_{U} und f_{O} sind somit drei Oktaven umfasst, welche demnach dreimal sechs Ganztonschritte, entsprechend 72 Vierteltonschritte beinhalten. Zusammen mit den Eckfrequenzen selbst sind somit 73 Tonhöhen abzudecken, welche nacheinander, jedoch lediglich in absteigender Frequenzrichtung, im Schaubild also von rechts nach links, durchlaufen werden.

Ebenfalls ist in dem Schaubild eine Reizschwelle 1 dargestellt, welche den Übergang von einer nicht spürbaren hin zu einer spürbaren Reizung des mit dem genannten Wechselstrom beaufschlagten Muskel- und Nervengewebe darstellt und somit eine natürlich gegebene Schwelle ist. Eine spürbare Reizung findet dabei im überschwelligen Bereich 2, eine nicht spürbare "Reizung" - also eigentlich bei richtigem Verständnis keine Reizung mehr - findet hingegen im unterschwelligen Bereich 3 statt. Eine Überquerung dieser Reizschwelle 1 beim Durchlaufen einer Amplituden-Frequenz-Kennlinie führt demnach vom unterschwelligen Bereich 3 kommend zur Kontraktion, vom überschwelligen Bereich 2 kommend hingegen zur Erschlaffung eines mit dem entsprechenden Wechselstrom beaufschlagten Muskels. Im Rahmen der Erfindung wird auf ein Durchlaufen der Kennlinien in Richtung aufsteigender Frequenz verzichtet und die entsprechenden Durchläufe gegen Null verkürzt. Dies hat den Vorteil, daß eine Modulation der gewählten Frequenz f mit einer Modulationsfrequenz etwa von 20 Hz möglich ist, wobei gleichzeitig alle 73 Vierteltonhöhen angeboten werden können. Die Modulationsfrequenz ist mit 20 Hz möglichst nahe am natürlichen Vorbild gewählt, so daß sich eine Muskelkontraktion für eine etwa zu behandelnde Person so angenehm wie möglich darstellt.

Figur 2 zeigt eine weitere Kurvenschar von Frequenz-Amplituden-Kennlinien, wobei in dieser Konfiguration der Sammelpunkt 4 im überschwelligen Bereich 2 liegt. Demnach schneiden hier sämtliche Frequenz-Amplituden-Kennlinien die Reizschwelle 1.

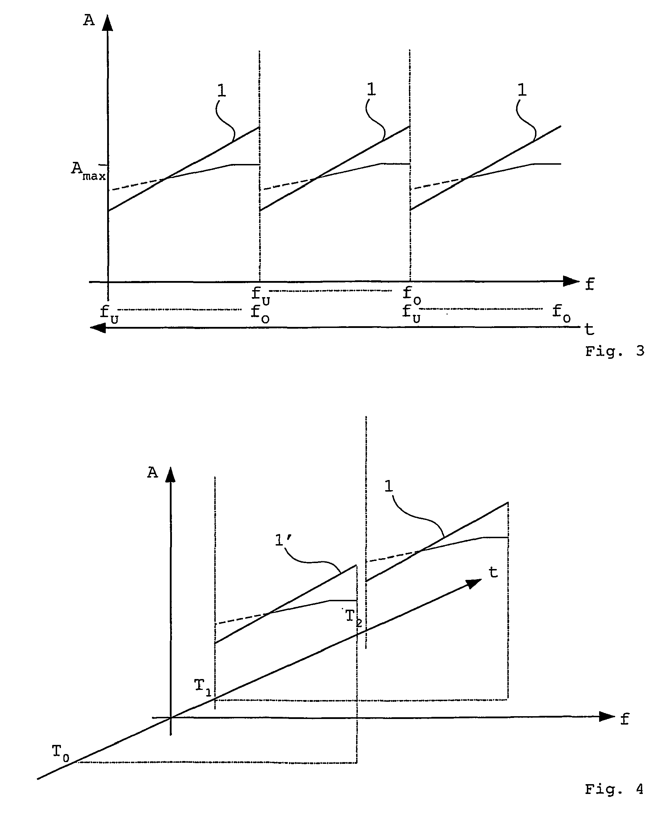

Die Figuren 3 und 4 bringen in den bloßen Frequenz- und Amplitudengang zusätzlich den Zeitaspekt hinein, indem die Zeit t in einer zusätzlichen Achse aufgetragen ist.

In Figur 3 verläuft die Zeitachse gegenparallel zur Frequenzachse, so daß eine Abfolge von mehreren Durchläufen gezeigt werden kann. Die zwischen den einzelnen Durchläufen von der unteren Eckfrequenz f_{U} bis zur oberen Eckfrequenz f_{O} einschiebbaren Pausen können alternativ durch ein Halten der Amplitude A auf einem aktuellen Wert oder auf einem maximalen Wert Aₘₐₓ bzw. durch ein Zurückgehen und Halten eines Amplitudenwerts von Null gefüllt werden.

Eine andere Ansicht desselben Sachverhalts zeigt Figur 4, in welcher ein dreidimensionales Koordinatensystem mit einer eigenen Zeitachse enthalten ist. Auf der Zeitachse sind hierbei der Beginn einer ersten Periode T₁ und einer zweiten Periode T₂ aufgetragen.

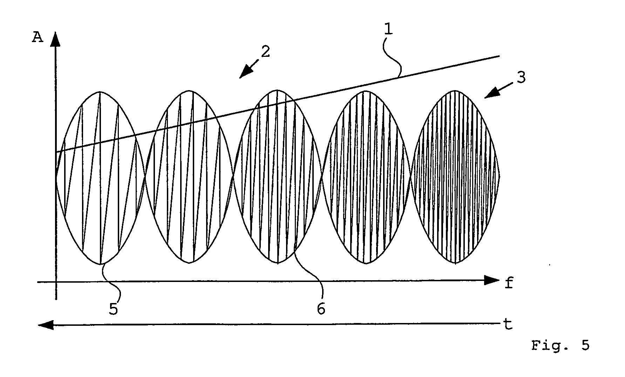

Figur 5 zeigt einen möglichen Therapiervorgang, welcher vorsieht, die Trägerfrequenz mit einer Modulationsfrequenz zu überlagern. Die Trägerkurve 6 führt dabei die Vierteltonschritte aus, welche eine Reizung des behandelten Muskel- und Nervengewebes verursachen, während die Hüllkurve mit einer Frequenz von 20 Hz eine Modulation der Hochton-Trägerfrequenzen in eine natürliche, körpereigene Frequenz veranschaulicht.

Somit ist vorstehend eine Vorrichtung zur elektrotherapeutischen Behandlung von Muskel- und Nervengewebe beschrieben, welche eine lückenlose Behandlung unter bevorzugten Bedingungen ermöglicht, indem auf eine Phase ansteigender Frequenz zugunsten einer Phase absteigender Frequenz zumindest teilweise verzichtet wird.

### B E Z U G S Z E I C H E N L I S T E

- 1,1': Reizschwelle
- 2: überschwelliger Bereich
- 3: unterschwelliger Bereich
- 4: Sammelpunkt
- 5: Hüllkurve
- 6: Trägerkurve
- T₁: Beginn Erste Periode
- T₂: Beginn Zweite Periode
- f: Frequenz
- f_{U}: untere Eckfrequenz
- fo: obere Eckfrequenz
- A: Amplitude
- Aₘₐₓ: Amplitudengrenzwert
- t: Zeit

## Patentansprüche

1. Vorrichtung zur elektrotherapeutischen Behandlung von Muskel- und Nervengewebe, mit einem Signalgeber zum Generieren von Wechselstrom definierter Frequenz und Amplitude sowie wenigstens zwei Elektroden zur Beaufschlagung des Muskel- und Nervengewebes mit diesem Wechselstrom, wobei der Signalgeber periodisch eine simultane Modulation von Frequenz (f) und Amplitude (A) in einer Phase ansteigender Frequenz und einer Phase absteigender Frequenz erzeugt, wobei eine Modulation der Frequenz (f) mit einer Modulationsfrequenz aus dem Frequenzbereich von 0,1 Hz bis 200 Hz erfolgt und die modulierte Frequenz, d.h. die Trägerfrequenz, ein Frequenzband von 4 kHz bis 32 kHz in diskreten Vierteltonschritten derart durchläuft, dass jeder Vierteltonschritt in einer Modulationsperiode zumindest zweimal angeboten wird, wobei die Phase ansteigender Frequenz zugunsten der Phase absteigender Frequenz auf zumindest näherungsweise Null verkürzt ist.

2. Vorrichtung gemäß Anspruch 1, **dadurch gekennzeichnet, dass** eine Reizschwelle (1,1') definiert ist, welche in der Phase ansteigender Frequenz von einem überschwelligen (2) in einen unterschwelligen Bereich (3) und in der Phase absteigender Frequenz von einem unterschwelligen (3) in einen überschwelligen Bereich (2) bezüglich der Reizschwelle (1,1') durchkreuzt wird.

3. Vorrichtung gemäß Anspruch 2, **dadurch gekennzeichnet, dass** eine Amplituden-Frequenz-Kennlinie durch den Signalgeber so einstellbar ist, dass diese die Reizschwelle (1,1') in spitzem Winkel schneidet.

4. Vorrichtung gemäß einem der vorhergehenden Ansprüche, **dadurch gekennzeichnet, dass** Frequenz (f) und Amplitude (A) in einem proportionalen Verhältnis zueinander gewählt sind.

5. Vorrichtung gemäß einem der vorhergehenden Ansprüche, **dadurch gekennzeichnet, dass** die in das Muskel- und Nervengewebe eingespeiste Leistung, vorzugsweise mittels eines wählbaren Amplitudengrenzwertes (Aₘₐₓ), begrenzt ist.

6. Vorrichtung gemäß Anspruch 5, **dadurch gekennzeichnet, dass** eine absolute obere Begrenzung der eingespeisten Leistung bei 5000 mW liegt.

7. Vorrichtung gemäß einem der vorhergehenden Ansprüche, **dadurch gekennzeichnet, dass** ein Therapiervorgang programmierbar ist.

8. Vorrichtung gemäß Anspruch 7, **dadurch gekennzeichnet, dass** die Steigung der Amplituden-Frequenz-Kennlinie im Laufe eines Therapiervorgangs anhebbar oder absenkbar ist.

9. Vorrichtung gemäß einem der Ansprüche 7 oder 8, **dadurch gekennzeichnet, dass** zwischen den Reizphasen Pausen dadurch einfügbar sind, das Frequenz (f) und/oder Amplitude (A) zwischendurch auf Null zurückgehen oder dass die, vorzugsweise maximale, Amplitude (A) bei der oberen Eckfrequenz (f_{O}) gehalten wird.

10. Vorrichtung gemäß einem der Ansprüche 7 bis 9, **dadurch gekennzeichnet, dass** der Ablauf des Therapiervorgangs von der paradoxen Stimulation über die synergistische hin zur vertikalen Stimulation verläuft.

11. Vorrichtung gemäß einem der vorhergehenden Ansprüche, **dadurch gekennzeichnet, dass** die Modulationsfrequenz ihrerseits moduliert ist.

12. Vorrichtung gemäß einem der vorhergehenden Ansprüche, **dadurch gekennzeichnet, dass** die einzelnen Parameter der Programmierung zumindest teilweise wählbar sind.

## Claims

1. A device for the electrotherapeutic treatment of muscle and nerve tissue, having a signal generator for generating alternating current of defined frequency and amplitude and at least two electrodes for exposing the muscle and nerve tissue to this alternating current, the signal generator periodically generating a simultaneous modulation of frequency (f) and amplitude (A) in a rising frequency phase and a falling frequency phase, wherein a modulation of frequency (f) proceeds with a modulation frequency from the frequency range from 0.1 Hz to 200 Hz and the modulated frequency, i.e. the carrier frequency, passes through a frequency band from 4 kHz to 32 kHz in discrete quarter-tone steps in such a manner that each quarter-tone step is offered at least twice in a modulation period, the rising frequency phase being shortened to at least approximately zero in favour of the falling frequency phase.

2. A device according to claim 1, **characterised in that** a stimulus threshold (1, 1') is defined which, in the rising frequency phase, is crossed from a super-threshold (2) into a sub-threshold range (3) and, in the falling frequency phase, from a sub-threshold (3) into a super-threshold range (2) relative to the stimulus threshold (1, 1').

3. A device according to claim 2, **characterised in that** an amplitude-frequency characteristic curve may be adjusted by the signal generator in such manner that said curve intersects the stimulus threshold (1, 1') at an acute angle.

4. A device according to any one of the preceding claims, **characterised in that** frequency (f) and amplitude (A) are selected in a ratio proportional to one another.

5. A device according to any one of the preceding claims, **characterised in that** the power introduced into the muscle and nerve tissue is limited, preferably by means of a selectable amplitude limit value (Aₘₐₓ).

6. A device according to claim 5, **characterised in that** there is an absolute upper limit for introduced power at 5000 mW.

7. A device according to any one of the preceding claims, **characterised in that** a therapeutic procedure is programmable.

8. A device according to claim 7, **characterised in that** the gradient of the amplitude-frequency characteristic curve may be raised or lowered over the course of a therapeutic procedure.

9. A device according to any either of claims 7 or 8, **characterised in that** pauses may be inserted between the stimulus phases by the frequency (f) and/or amplitude (A) declining to zero between times or by the, preferably maximum, amplitude (A) being maintained at the upper cut-off frequency (f₀).

10. A device according to any one of claims 7 to 9, **characterised in that** the course of the therapeutic procedure runs from paradoxical stimulation via synergistic stimulation to vertical stimulation.

11. A device according to any one of the preceding claims, **characterised in that** the modulation frequency is itself modulated.

12. A device according to any one of the preceding claims, **characterised in that** the individual parameters of the programming are at least partially selectable.

## Revendications

1. Dispositif de traitement électrothérapeutique de tissus musculaires et nerveux, doté d'un émetteur de signal pour générer un courant alternatif de fréquence et amplitude définies, et d'au moins deux électrodes pour appliquer au tissu musculaire et nerveux ce courant alternatif, l'émetteur de signal générant périodiquement une modulation simultanée de fréquence (f) et d'amplitude (A) dans une phase de fréquence montante et une phase de fréquence descendante, où une modulation de la fréquence (f) s'effectue avec une fréquence de modulation dans la gamme de fréquences allant de 0,1 Hz à 200 Hz et où la fréquence modulée, c.-à-d. la porteuse, parcourt une bande de fréquences allant de 4 kHz à 32 kHz en intervalles discrets de quart de ton de telle sorte que chaque intervalle de quart de ton est proposé au moins deux fois dans une période de modulation, la phase de fréquence montante étant réduite à approximativement zéro au profit de la phase de fréquence descendante.

2. Dispositif selon la revendication 1, **caractérisé en ce qu'**un seuil de stimulation (1, 1') est défini, lequel est traversé dans la phase de fréquence montante d'une zone supra-seuil (2) vers une zone infra-seuil (3) et dans la phase de fréquence descendante d'une zone infra-seuil (3) vers une zone supraliminaire (2) par rapport au seuil de stimulation (1, 1').

3. Dispositif selon la revendication 2, **caractérisé en ce qu'**une caractéristique amplitude-fréquence peut être réglée par l'émetteur de signal de façon à ce que celle-ci coupe le seuil de stimulation (1, 1') à angle aigu.

4. Dispositif selon l'une des revendications précédentes, **caractérisé en ce que** la fréquence (f) et l'amplitude (A) sont choisies selon un rapport proportionnel entre elles.

5. Dispositif selon l'une des revendications précédentes, **caractérisé en ce que** la puissance injectée dans le tissu musculaire et nerveux est limitée, de préférence au moyen d'une valeur limite d'amplitude que l'on peut choisir.

6. Dispositif selon la revendication 5, **caractérisé en ce qu'**une limitation absolue supérieure de la puissance injectée est de l'ordre de 5000 mW.

7. Dispositif selon l'une des revendications précédentes, **caractérisé en ce que** le processus thérapeutique est programmable.

8. Dispositif selon la revendication 7, **caractérisé en ce que** la pente de la caractéristique amplitude-fréquence peut être augmentée ou abaissée au cours d'un processus thérapeutique.

9. Dispositif selon l'une des revendications 7 ou 8, **caractérisé en ce que** des pauses peuvent être insérées entre les phases de stimulation, en ramenant entre-temps la fréquence (f) et/ou l'amplitude (A) à zéro ou en maintenant l'amplitude (A) de préférence maximale proche de la fréquence limite supérieure (f₀).

10. Dispositif selon l'une des revendications 7 à 9, **caractérisé en ce que** le déroulement du processus thérapeutique va de la stimulation paradoxale à la stimulation verticale en passant par la stimulation synergiste.

11. Dispositif selon l'une des revendications précédentes, **caractérisé en ce que** la fréquence de modulation est modulée de son côté.

12. Dispositif selon l'une des revendications précédentes, **caractérisé en ce que** les différents paramètres de la programmation peuvent être choisis au moins partiellement.
